# EUROPEAN PATENT APPLICATION

(11) **EP 4 230 655 A2**
(43) Date of publication of application: **23.08.2023**
(21) Application number: 23183390.6
(22) Date of filing: 10.09.2020
(51) Int. Cl.: C07K 16/28, A61P 25/00, A61P 25/28

(54) **TREATMENT OF RMS BY SWITCHING THERAPY**

(30) Priority: 11.09.2019 EP 19196789; 30.01.2020 EP 20154736
(62) Divisional of application: 20785440.7
(71) Applicant: Novartis AG, 4056 Basel (CH)
(72) Inventor: RAMANATHAN, Krishnan, 4002 Basel (CH); HAERING, Dieter Adrian, 4002 Basel (CH); BAGGER, Morten, 4002 Basel (CH); MERSCHHEMKE, Martin, 4002 Basel (CH); ZIEHN, Marina, 4002 Basel (CH); PINGILI, Ratnakar, Hanover, 07936-1080 (US)
(74) Representative: Carpmaels & Ransford LLP

(57) **Abstract**

The present invention relates to the anti-CD20 monoclonal antibody ofatumumab for use in the treatment or prevention of relapsing multiple sclerosis, wherein ofatumumab is used in a patient who has been treated with a disease-modifying therapy other than ofatumumab.

## Description

### FIELD

The present invention relates to the anti-CD20 monoclonal antibody ofatumumab for use in the treatment or prevention of relapsing multiple sclerosis, wherein ofatumumab is used in a patient who had been treated with a disease-modifying therapy other than ofatumumab.

### BACKGROUND

Multiple Sclerosis (MS) is a chronic, immune-mediated disease of the central nervous system characterized by inflammation, demyelination and axonal/neuronal destruction, ultimately leading to severe disability. Although there is no cure for the disease, a variety of disease-modifying therapies (DMT) are available which usually slow down diseases progression.

DMT includes the administration of a disease-modifying drug (DMD). Examples of approved drugs for the treatment of MS are glatiramer acetate, ocrelizumab, cladribine, fingolimod, natalizumab, teriflunomide, mitoxantrone or dimethyl fumarate (DMF).

While these DMTs usually significantly reduce relapse rates and MRI disease activity and thus delay the time to disability worsening, generally (severe) adverse events may be associated with each of these DMTs. For example, natalizumab may lead to an increased risk of a fatal opportunistic infection (i.e. progressive multifocal leukoencephalopathy or PML). Fingolimod may be associated with S 1P-related safety risks, e.g. bradyarrhythmias upon treatment initiation, macular edema, hypertension and liver transaminase elevations.

Given that existing drugs for the treatment of MS have associated risks, there remains a need to identify ways to reduce, minimize or overcome these risks. In particular, there remains a need to address these and other risks that arise when patients are discontinuing an earlier therapy, e.g. when patients are transitioning from an earlier disease-modifying therapy (DMT) such as fingolimod or DMF to another DMT.

This is because the treatment of multiple sclerosis (MS) may span decades and, therefore, the need often arises to make changes to the treatment plan in order to accommodate changing circumstances. Switching drugs, or the discontinuation of immunomodulatory agents altogether, may leave patients vulnerable to relapse or disease progression. In some cases, severe MS disease activity is noted clinically and on MRI after treatment withdrawal. When this disease activity is disproportionate to the pattern observed prior to treatment initiation, patients are said to have experienced rebound.

Pregnancy planning is a frequent reason for DMT discontinuation. DMTs have been associated with birth defects in animal studies, and it is recommended that fingolimod be stopped prior to conception.

Several of the approved MS drugs are implicated in rebound, i.e. in a severe disease reactivation after the withdrawal of DMT that exceeds a patient's pre-DMT baseline. For example, the problem of rebound is discussed for natalizumab and fingolimod (Barry B. et al.: Fingolimod Rebound: A Review of the Clinical Experience and Management Considerations. Neurol Ther (2019) 8:241-250). Thus, there is a need to reduce, minimize or overcome the risk of rebound after discontinuation of DMTs such as natalizumab or fingolimod.

Since, moreover, rebound has been reported in patients who showed breakthrough disease under treatment with a DMT such as fingolimod, there is a need to improve treatment strategies for patients with breakthrough disease.

Therefore, the problem underlying the present invention is to provide improved treatment strategies for MS patients necessitating treatment optimization. For example, MS is relapsing-remitting MS (RRMS) and the treatment to be optimized is a DMT. Reasons for treatment optimization may include adverse effects, treatment failure, breakthrough disease, disease progression, comorbidities, life cycle events such as pregnancy and lactation, and/or evolving patient preferences.

As a consequence, any interruption or change in treatment, such as when switching therapies, leaves a patient with active MS vulnerable to relapses.

Reasons for treatment interruption or change include adverse effects, treatment failure, disease progression, comorbidities, life cycle events such as pregnancy and lactation, and evolving patient preferences. As fulminant MS rebound events resembling immune reconstitution inflammatory syndrome (IRIS) have been reported with the withdrawal of treatment in MS, it is important for the clinician to be aware of those circumstances that put patients at risk of severe disease reactivation.

Thus, there is a need to reduce or minimize this vulnerability if treatment is interrupted or changed.

Despite the above illustrated existing need, reliable evidence-based recommendations on successful transitioning between DMTs are not available. The risk of additive immune system effects when switching from drugs with immune effects such as fingolimod or DMF needs to be considered and balanced against the risk of rebound or recurrent disease activity.

In the art, B-cell depletion with rituximab or ocrelizumab has been suggested as a treatment strategy to manage relapses and rebound after DMT cessation. In particular, rituximab has been suggested as a treatment strategy to manage rebound after fingolimod cessation. In a series published by Hatcher et al., however, one case featured clinical worsening 1 day after rituximab infusion (Hatcher et al., Rebound syndrome in patients with multiple sclerosis after cessation of fingolimod treatment, JAMA Neurol. 2016;73(7):790-4.). Two other cases featured persistent Gd-enhancing lesions despite treatment with steroids and rituximab.

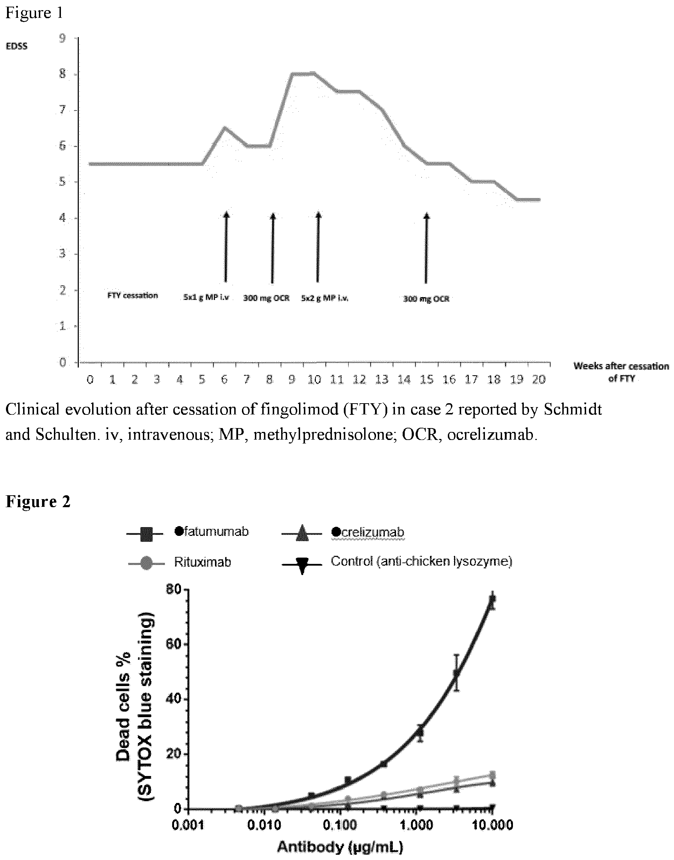

In a separate report, two patients with rebound disease after fingolimod cessation were noted to have clinical worsening and new Gd-enhancing MRI lesions 1 week after initiation of ocrelizumab (Schmidt S, Schulten T., Severe rebound after cessation of fingolimod treated with ocrelizumab with coincidental transient aggravation: report of two cases. Ther Adv Neurol Disord. 2019; 12:1-6.). Two patients exhibited significant Expanded Disability Status Scale (EDSS) progression after administration of ocrelizumab despite immune reconstitution more than 3 months after fingolimod withdrawal. This is shown for one of the patients in Fig.1.

Both patients with highly active RRMS showed breakthrough disease under treatment with fingolimod, necessitating treatment optimization, and subsequently developed clinical and MRI features of severe rebound after cessation of fingolimod. Rebound in both patients occurred as early as 4-6 weeks after discontinuation of fingolimod. Schmidt and Schulten suggested that rebound might be explained by the release of TH17 T cells trapped in the secondary lymphoid organs invading the central nervous system. Pharmacodynamic data showed rapid recovery of lymphocyte counts starting several days after treatment cessation. Since ocrelizumab depletes pre-B cells and mature and memory B cells, it disrupts regulatory signals transmitted by B cells. For example, B cells produce regulatory IL-10-inhibiting differentiation of pathogenic Th1 and Th17 cells and secrete IL-35, 14, 15, a recently discovered regulatory cytokine of critical importance during autoimmune attacks. Regulatory B cells also secrete TGF-β. Given the regulatory function of certain B-cell subsets, it appears plausible that the removal of these B cells from the peripheral immune system even several weeks after cessation of fingolimod treatment might have contributed to the secondary deterioration in both patients.

Thus, having regard to the prior art, B-cell depletion has not emerged as a promising avenue for the management of relapses and rebound after DMT (e.g. natalizumab or fingolimod) cessation.

### SUMMARY OF THE INVENTION

Despite this teaching away in the prior art, the present invention surprisingly provides a treatment strategy that effectively reduces the risk of disease reactivation, relapses and rebound by administering another B-cell depleting agent.

In particular, it has been completely surprisingly found that B-cell depletion with ofatumumab provides a powerful and efficacious treatment strategy to manage severe relapses and rebound after DMT (e.g. natalizumab or fingolimod) cessation. This treatment strategy is most efficient when ofatumumab therapy is commenced within 0-6 months.

As mentioned above, there is a need to reduce, minimize or overcome the risk of rebound after discontinuation of DMTs such as natalizumab or fingolimod. This need is surprisingly met by the present invention.

Although the modes of action of natalizumab and fingolimod are distinct, the "anti-trafficking" strategy shared by both natalizumab and fingolimod reduces CNS entry of lymphocytes, and may help explain why a rebound phenomenon may occur when these drugs are stopped. Though further study is needed, fingolimod has a more complex mode of action than a simple anti-trafficking function, likely augmenting beneficial processes while preventing disadvantageous processes within the immune system. In a small analytic study of messenger RNA expression in peripheral blood CD4+ cells, for example, fingolimod treatment was associated with altered transcription levels of 890 different genes. Many of these genes affect cytokine secretion, Toll-like receptor expression, and cell adhesion molecules that may be involved in T cell functions that suppress inflammation and autoimmunity. Surprisingly, it has been found that ofatumumab reduces said rebound phenomenon although it does not seem to directly affect the processes that have been believed to cause rebound.

Unexpectedly, ofatumumab has the potential to reduce the risk of rebound in patients with breakthrough disease activity while on fingolimod or another DMT different from ofatumumab.

Without being bound by theory, it is believed that ofatumumab spares regulatory signals (e.g. transmitted by B cells), maybe even (a subset of) regulatory T or B cells per se. This is evidenced by Theil et al., 2019, Imaging Mass Cytometry and Single-Cell Genomics Reveal Differential Depletion and Repletion of B-Cell Populations Following Ofatumumab Treatment in Cynomolgus Monkeys. Frontiers in Immunology (2019), Volume 10: 1-11. Therefore, it is plausible that administration of ofatumumab after cessation of a DMT treatment such as fingolimod treatment significantly reduces the risk of a secondary deterioration (e.g. rebound after breakthrough disease activity).

Taken together, rebound after DMT discontinuation (e.g. fingolimod discontinuation) may be aggravated by the initiation of treatment with another DMT such as ocrelizumab. Thus, there are potential pitfalls and undesirable consequences of sequential application of potent immunomodulatory and immunosuppressive drugs. In particular, ocrelizumab may complicate recovery of rebound after cessation of fingolimod. Therefore, it has been completely surprising that ofatumumab does not result in such complications or at least significantly reduces the risk thereof.

Experimental withdrawal of fingolimod resulted in overexpression of lymphocytic S1P1 receptors leading to lymphocyte egress from lymph nodes and an increase in severity of relapse symptoms. Withdrawal of fingolimod might also result in astrocytic overexpression of S1P1 and a downstream inflammatory response, possibly mediated by NF-κB activation and release of inflammatory cytokines and nitric oxide. Since overexpression of S1P and/or its receptor might also play a role in the etiology of breakthrough disease, it is plausible that ofatumumab is suitable to treat both breakthrough disease and relapse syndromes such as those that occur as a result of rebound phenomena.

Further, it has been completely surprising that B-cell depletion with ofatumumab provides an efficacious strategy to avoid undesirable risks of DMT therapy in pregnant women or prior to pregnancy.

Hence, a subject of the present invention is ofatumumab for use in the treatment or prevention of relapsing multiple sclerosis, wherein ofatumumab is used in a patient who has been treated with a disease-modifying therapy (DMT) other than ofatumumab. DMT is defined below. All preferred embodiments illustrated below also apply for the use of the invention.

A further subject of the invention is a method for treating or preventing relapsing multiple sclerosis, wherein the method comprises administration of ofatumumab to a patient suffering from relapsing multiple sclerosis, said patient having taken a disease-modifying therapy other than ofatumumab. All preferred embodiments illustrated below also apply for the method of the invention.

A further subject of the invention is ofatumumab for producing a medicament for use in the treatment or prevention of relapsing multiple sclerosis, wherein the medicament is used in a patient who has been treated with a disease-modifying therapy other than ofatumumab. All preferred embodiments illustrated below also apply for this subject of the invention.

### DETAILED DESCRPTION OF THE INVENTION

In a preferred embodiment, the disease-modifying therapy other than ofatumumab is an earlier DMT. This means, in a preferred embodiment the patient is switched from an earlier DMT to ofatumumab. In other words, the invention concerns ofatumumab for use in the treatment or prevention of relapsing multiple sclerosis, wherein ofatumumab is used in a patient transitioning from a disease-modifying therapy.

In an embodiment of the invention, ofatumumab is not administered to patients having an active HBV infection.

In a preferred embodiment of the invention, the disease-modifying therapy other than ofatumumab is discontinued before initiation of ofatumumab administration.

In a preferred embodiment of the invention, ofatumumab administration is started before the half-life of the drug used in the disease-modifying therapy other than ofatumumab has been reached in the patient.

In a preferred embodiment of the present invention, ofatumumab therapy is commenced immediately after discontinuation of an earlier DMT (such as glatiramer acetate, ocrelizumab, cladribine, fingolimod, natalizumab, teriflunomide, mitoxantrone or dimethyl fumarate). In this context, immediately means one week, preferably three days, more preferably two days, more preferably one day, most preferred within 12 hours after discontinuation of an earlier DMT (such as glatiramer acetate, ocrelizumab, cladribine, fingolimod, natalizumab, teriflunomide, mitoxantrone or dimethyl fumarate).

In another embodiment of the present invention, ofatumumab therapy is commenced before discontinuation of an earlier DMT (such as glatiramer acetate, ocrelizumab, cladribine, fingolimod, natalizumab, teriflunomide, mitoxantrone or dimethyl fumarate). For example, the earlier DMT may be continued until a loading dose regimen of ofatumumab has been administered. A loading dose regimen may comprise subcutaneous (s.c.) injections of 20 mg ofatumumab at day 1, day 7 and day 14 of the dosage regimen; alternatively, a loading dose regimen may comprise s.c. injections of 20 mg ofatumumab at day 0, day 7 and day 14 of the dosage regimen.

Ofatumumab therapy and an earlier DMT (such as glatiramer acetate, ocrelizumab, cladribine, fingolimod, natalizumab, teriflunomide, mitoxantrone or dimethyl fumarate) may proceed in parallel. That is, the ofatumumab administration is started before the administration of the earlier DMT has stopped. For example, said therapies may proceed in parallel for 1 day, 3 days, one week, two weeks or one month.

In an embodiment of the present invention, ofatumumab therapy is commenced when the disease-modifying therapy other than ofatumumab (e.g. an earlier DMT) has lost efficacy. For example, the medication used in the earlier DMT may be washed out. Preferably, the medication is a DMD. Thus, ofatumumab therapy may be commenced when the earlier DMT's medication (e.g. DMD) is washed out. In this case, the earlier DMT is the first DMT and ofatumumab is the second DMT. In a preferred embodiment, the medication of the first DMT is considered to be washed out if 25%, preferably 50%, more preferably 75%, even more preferably 85%, most preferably 95% of the medication's half-life have elapsed until the last dose of the first DMT's medication has been administrated before discontinuation of the first DMT.

In an alternative embodiment, the first DMT's medication is considered to be washed out if only 30%, preferably 20%, more preferably 10%, even more preferably 5%, most preferably 2.5% or less of the amount administered as the last dose of the first DMT's medication can be detected in samples (e.g. blood or serum) from the patient. In a particularly preferred embodiment, said amount can be replaced by Cₘₐₓ, i.e. the maximum (or peak) serum concentration that the first DMT's medication achieves in serum after the last dose of the first DMT's medication has been administrated before discontinuation of the first DMT.

In an embodiment of the present invention, ofatumumab therapy is commenced within 0-6 months, more preferably 1-5 months, more preferably within 2-4 months, and even more preferably within the third month after stopping the earlier disease-modifying therapy (e.g. fingolimod). More specifically, ofatumumab therapy may be commenced between 4 and 16 weeks, more preferably between 5 and 15 weeks, more preferably between 6 and 14 weeks, more preferably between 7 and 13 weeks, more preferably between 8 and 12 weeks, more preferably between 9 and 11 weeks, more preferably about 10 weeks after stopping the earlier DMT, e.g. fingolimod. Alternatively, ofatumumab therapy may be commenced before 10 weeks, more preferably before 9 weeks, more preferably before 8 weeks, more preferably before 7 weeks, more preferably before 6 weeks, more preferably before 5 weeks, more preferably before 4 weeks after stopping the earlier DMT, e.g. fingolimod.

Alternatively, ofatumumab therapy is commenced between 3 and 18 weeks, more preferably between 4 and 17 weeks, more preferably between 5 and 16 weeks, more preferably between 6 and 15 weeks, more preferably between 7 and 14 weeks, more preferably between 8 and 13 weeks, more preferably between 9 and 12 weeks, more preferably between 10 and 11 weeks, more preferably about 10.5 weeks after earlier DMT cessation.

In a preferred embodiment of the present invention, the ofatumumab administration is started without a wash-out period.

In a preferred embodiment of the present invention, the patient has been treated with the disease-modifying therapy other than ofatumumab for at least 6 months, more preferably at least 7 months, more preferably at least 8 months, more preferably at least 9 months, more preferably at least 10 months, more preferably at least 11 months, more preferably at least 12 months. The patient may have been treated with the DMT other than ofatumumab for example for up to 10 years, up to 8 years, up to 6 years, up to 4 years or up to 2 years.

In a particularly preferred embodiment, the use of ofatumumab according to the invention prevents or reduces rebound. Rebound is defined below. Rebound may manifest within 0-6 months, or 1-5 months, or within 2-4 months, and even more preferred within the third month after stopping the other disease-modifying therapy (e.g. fingolimod). More specifically, rebound may manifest between 4 and 16 weeks, or between 5 and 15 weeks, or between 6 and 14 weeks, or between 7 and 13 weeks, or between 8 and 12 weeks, or between 9 and 11 weeks, or about 10 weeks after stopping the other disease-modifying therapy (e.g. fingolimod). Alternatively, rebound may manifest between 3 and 18 weeks, or between 4 and 17 weeks, or between 5 and 16 weeks, or between 6 and 15 weeks, or between 7 and 14 weeks, or between 8 and 13 weeks, or between 9 and 12 weeks, or between 10 and 11 weeks, more preferably about 10.5 weeks after cessation of the other or earlier disease-modifying therapy (e.g. fingolimod).

In a preferred embodiment of the present invention the disease-modifying therapy other than ofatumumab lacks efficacy. A lack of efficacy is present for example if a patient who is on the disease-modifying therapy (DMT) shows signs of disease activity, such as relapses or lesions. Lack of efficacy can be defined as not stopping or not appropriately slowing down disease progression. In other words, the present invention is directed to use of ofatumumab for treating non-responders to the earlier DMT.

In a preferred embodiment, ofatumumab is administered to patients with suboptimal response to DMT therapy, e.g. to anti-CD20 therapy. In a preferred embodiment, the suboptimal response occurred within the previous 6 months. In a preferred embodiment, suboptimal response might be characterized by relapse, ≥2 active gadolinium-enhancing [Gd+] lesions, any new/enlarging T2 lesions and/or clinical worsening.

In a preferred embodiment of the present invention ofatumumab is administered after the detection of at least one Gd+ lesion. The term Gd+ lesion is defined below.

In a preferred embodiment of the present invention ofatumumab is administered after the detection of new or enlarging T2 lesions. The term T2 lesion is described below.

In an embodiment of the present invention the patient developed breakthrough disease due to treatment with the other disease-modifying therapy, e.g. due to the treatment with the earlier DMT. In an alternative embodiment of the present invention rebound or recurrent disease activity is avoided.

Generally, breakthrough disease is evidenced by one or more clinically reported relapses or one or more signs of MRI activity, wherein the MRI activity comprises Gd+ enhancement and/or new or enlarging T2 lesions. Reference is made to the definition of breakthrough disease as given below.

Breakthrough disease may manifest within 0-6 months, or 1-5 months, or within 2-4 months, and even more preferred within the third month before stopping the other disease-modifying therapy (e.g. fingolimod). More specifically, breakthrough disease may manifest between 4 and 16 weeks, or between 5 and 15 weeks, or between 6 and 14 weeks, or between 7 and 13 weeks, or between 8 and 12 weeks, or between 9 and 11 weeks, or about 10 weeks before stopping the other disease-modifying therapy (e.g. fingolimod). Alternatively, breakthrough disease may manifest between 3 and 18 weeks, or between 4 and 17 weeks, or between 5 and 16 weeks, or between 6 and 15 weeks, or between 7 and 14 weeks, or between 8 and 13 weeks, or between 9 and 12 weeks, or between 10 and 11 weeks, more preferably about 10.5 weeks before cessation of the other disease-modifying therapy (e.g. fingolimod).

In an alternative preferred embodiment of the present invention, the patient lacks tolerability for the other or earlier disease-modifying therapy. Hence, in a preferred embodiment, ofatumumab is administered to a patient who discontinued the earlier DMT due to adverse events.

Preferably, a lack of tolerability relates to the presence of adverse events such as headache, dizziness, nausea, infections (such herpes zoster), macular edema, infusion-related reactions or recurrent infections.

In an embodiment of the present invention the patient has a history of one, two or three disease-modifying therapies other than ofatumumab.

The terms "two or three disease modifying therapies" preferably relates to two or three different drugs.

In a preferred embodiment of the invention the patient is neurologically stable within one month prior to the first administration of ofatumumab. The term "neurologically stable" is defined below.

In a preferred embodiment of the invention ofatumumab is administered after a relapse, which can also be referred to as "acute relapse". The term relapse is defined below.

Further, the thalamic volume can act as a marker associated with neurodegeneration. Azevedo et al. reported that thalamic atrophy is present early in disease, reflects several aspects of MS pathology including gray matter injury and correlates well with physical and cognitive impairment. Hence, thalamic volume has been proposed as a potentially attractive MRI metric associated with neurodegenerative features of MS. Azevedo et al. found that thalamic volume declined significantly faster in MS subjects compared to healthy controls (HC), with an estimated decline of -0.71% per year (95% confidence interval [CI] =-0.77% to -0.64%) in MS subjects and -0.28% per year (95% CI = -0.58% to 0.02%) in HC (p for difference = 0.007). The rate of decline was consistent throughout the MS disease duration and across MS clinical subtypes, see "Thalamic Atrophy in Multiple Sclerosis: A Magnetic Resonance Imaging Marker of Neurodegeneration throughout Disease", Ann Neurol. 2018 February; 83(2): 223-234. doi:10.1002/ana.25150.

In the present invention, it was unexpectedly found that administration of ofatumumab leads to an advantageous reduction of loss of thalamus volume. Reference is made to the experimental section below.

Hence, in a preferred embodiment of the present invention, ofatumumab is administered when loss of thalamus volume was not sufficiently reduced by the earlier disease-modifying treatment. Reducing the loss of thalamus volume by less than 30% or less than 25% or less than 20% compared to untreated baseline can be regarded as not sufficiently reduced. In this regard the untreated baseline loss can be regarded as 0.71% per year.

For example, ofatumumab can be administered if the administration of the earlier disease-modifying treatment did not reduce the loss of thalamus volume below 0.70% per year or below 0.65% per year or below 0.60% per year. Alternatively, ofatumumab can be administered if the administration of the earlier disease-modifying treatment did not reduce the loss of thalamus volume below 1.40% or below 1.3% or below 1.2% within 24 months.

In this regard a further subject of the present invention is ofatumumab for use in the treatment or prevention of relapsing multiple sclerosis, wherein ofatumumab reduces the loss of thalamus volume, preferably below 0.70% per year or below 0.65% per year or below 0.60% per year. For example, the loss can be reduced to 0.30% to 0.70% per year or 0.40% to 0.65% per year or to 0.45% to 0.60% per year. Alternatively, the present invention relates to ofatumumab for use in the treatment or prevention of relapsing multiple sclerosis, wherein ofatumumab reduces the loss of thalamus volume below 1.40% or below 1.3% or below 1.2% within 24 months. For example, the loss can be reduced to 0.6% to 1.40% or to 0.8% to 1.3% or to 0.9% to 1.2% within 24 months.

The MSIS-29 (see definition below) is a clinically useful and scientifically sound measure of the impact of MS from the patient's perspective suitable for clinical studies and epidemiological studies. It is considered a reliable, valid and responsive PRO (Patient Reported Outcomes) measure that complements other indicators of disease severity used to improve our understanding of the impact of MS.

In the present invention it was unexpectedly found that administration of ofatumumab leads to an advantageous reduction of the MS impact scale MSIS-29 as defined below. Reference is made to the experimental section below.

Hence, in a preferred embodiment of the present invention, ofatumumab is administered when reduction of the MSIS-29 score under the earlier disease-modifying treatment was not sufficiently achieved. Reducing the MSIS-29 score by less than 2.5 or less than 2.0 or less than 1.5 can be regarded as not sufficiently reduced.

For example, ofatumumab can be administered if the administration of the earlier disease-modifying treatment did not achieve a reduction of the MSIS-29 score by 1.5 or 2.0 or 2.5 within 24 months.

In this regard a further subject of the present invention is ofatumumab for use in the treatment or prevention of relapsing multiple sclerosis, wherein ofatumumab reduces the MSIS-29 score. Preferably, ofatumumab reduces the MSIS-29 score by at least 1.5, more preferably at least 2.0, still more preferably at least 2.5 within 24 months. The reduction might be up to 3.0 or 3.5 or 4.0.

In a preferred embodiment of the invention the patient has an EDSS score of 1 to 4 prior to the first administration of ofatumumab. EDSS means Expanded Disability Status Scale and is defined below.

In a preferred embodiment, ofatumumab can be administered irrespective of body weight, sex, age, race or baseline B-cell count. For example, it is preferred that a 35-year-old woman having a body weight of 60 kg receives the same dose as a 50-year old man having a body weight of 90 kg. In particular, body weight, sex, age, race or baseline B-cell count do not have a clinically meaningful effect on the pharmacokinetics of ofatumumab.

In a preferred embodiment, ofatumumab is administered to patients who discontinued earlier DMT, e.g. anti-CD20 therapy, because of side effects such as severe infusion-related reactions or recurrent infections.

Generally, the term DMT is known in the art and defined below. With regard to the present invention, examples of suitable DMTs are treatments with the following drugs: teriflunomide, leflunomide, dimethyl fumarate, fingolimod, natalizumab, rituximab, ocrelizumab, alemtuzumab, daclizumab, glatirameracetat and laquinimod.

In a preferred embodiment of the present invention the disease-modifying therapy other than ofatumumab is administered orally. Examples of suitable DMTs are teriflunomide, leflunomide, laquinimod, dimethyl fumarate and fingolimod.

In a particular preferred embodiment of the present invention the disease-modifying therapy other than ofatumumab is fingolimod. Preferably, fingolimod is administered in a dose of 0.5 mg once daily. In an alternative embodiment fingolimod is administered in a daily dose of 0.1 mg to 2.5 mg, for example 0.25 mg.

In a particular preferred embodiment of the present invention the disease-modifying therapy other than ofatumumab is dimethyl fumarate (DMF). Preferably, DMF is administered in a daily dose of 120 mg to 480 mg, in particular 480 mg.

In a particular preferred embodiment of the present invention the disease-modifying therapy other than ofatumumab is laquinimod. Preferably, laquinimod is administered in a daily dose of 0.2 to 1.0 mg, preferably 0.6 mg.

In a particular preferred embodiment of the present invention the disease-modifying therapy other than ofatumumab is teriflunomide. Preferably, teriflunomide is administered in a daily dose of 6 to 18 mg, preferably 14 mg.

In a preferred embodiment of the present invention the disease-modifying therapy other than ofatumumab is administered by injection. Examples of suitable DMTs are natalizumab, rituximab, ocrelizumab, alemtuzumab, daclizumab, and glatiramer acetate.

In a preferred embodiment of the present invention the disease-modifying therapy other than ofatumumab is natalizumab. Preferably, natalizumab is administered by intravenous injections every four weeks at a dose of 100 to 500 mg, preferably, 300 mg.

In a preferred embodiment of the present invention the disease-modifying therapy other than ofatumumab is daclizumab. Preferably, daclizumab is administered in a dose of 50 to 250 mg, preferably 150 mg s.c. once monthly.

In a preferred embodiment of the present invention the disease-modifying therapy other than ofatumumab is glatiramer acetate. Preferably, glatiramer acetate is administered in a dose of 20 mg/mL by s.c. injection once-daily regimen, or 40 mg/mL by s.c. injection 3-times-per-week.

In a preferred embodiment of the present invention the disease-modifying therapy other than ofatumumab is rituximab. Preferably, rituximab is administered in a dose of 500 or 1,000 mg every 6-12 months, in particular intravenously.

In a preferred embodiment of the present invention the disease-modifying therapy other than ofatumumab is ocrelizumab. Preferably, ocrelizumab is administered in a dose of 600 mg every 6 months, in particular intravenously.

Preferably, patients have been previously treated with at least 2, e.g. 2-5 consecutive courses of intravenous ocrelizumab or rituximab. The last dose may be administered e.g. 4-9 months before ofatumumab was administered.

According to the present invention, there is maintained efficacy of ofatumumab in patients with RMS transitioning from intravenous anti-CD20 therapies.

In a preferred embodiment ofatumumab is administered to patients with suboptimal response to anti-CD20 therapy in the previous 6 months (e.g. relapse, ≥2 active gadolinium-enhancing [Gd+] lesions, any new/enlarging T2 lesions, clinical worsening) and/or to patients or who discontinued anti-CD20 therapy because of adverse events, e.g. severe infusion-related reactions or recurrent infections.

In a preferred embodiment of the present invention the disease-modifying therapy other than ofatumumab is alemtuzumab. Preferably, alemtuzumab is administered in a dose of 12 mg/day, administered as intravenous infusion.

In a preferred embodiment of the present invention ofatumumab is administered at a dose of 10 to 30 mg every 4 weeks, preferably 20 mg every 4 weeks. Preferably, ofatumumab is administered by subcutaneous injection (s.c.).

In a preferred embodiment of the present invention ofatumumab is administered with a loading dose. The term loading dose is defined below. In a preferred embodiment three loading doses are administered. Preferably in week 0 and in week 1 and in week 2 after starting ofatumumab therapy. This means, the first loading dose in week 0 constitutes the start of therapy. In an alternative preferred embodiment three loading doses are administered on day 1, and on day 5-9, preferably on day 7, and on day 12 - 16, preferably on day 14, after starting ofatumumab therapy. This means, the first loading dose on day 1 constitutes the start of therapy.

In a preferred embodiment of the present invention the loading dose is 10 - 30 mg, preferably 20 mg ofatumumab.

The preferred dosage of ofatumumab is:
- initial dosing of 20 mg by subcutaneous injection at Weeks 0, 1 and 2, followed by
- subsequent dosing of 20 mg by subcutaneous injection once monthly starting at Week 4.

If an injection of ofatumumab is missed, it should preferably be administered as soon as possible without waiting until the next scheduled dose. Subsequent doses should be administered at the recommended intervals.

In an alternative embodiment of the present invention ofatumumab is administered without a loading dose.

In a preferred embodiment of the present invention a premedication is administered to the patient before the first dose of ofatumumab is administered. Preferably, the premedication comprises acetaminophen, antihistamines and/or steroids. Methylprednisolone may be a preferred steroid. 100 mg iv may be a preferred dose. Preferably, the premedication is administered 30 to 60 minutes prior to ofatumumab injection.

In a particular preferred embodiment, no premedication is administered prior to the first dose of ofatumumab.

In a preferred embodiment of the invention ofatumumab is administered for the treatment of relapsing forms of multiple sclerosis (MS), to include clinically isolated syndrome, relapsing-remitting disease and active secondary progressive disease, preferably in adults.

In a preferred embodiment of the present invention relapsing multiple sclerosis is selected from relapsing remitting multiple sclerosis (RRMS) and secondary progressive multiple sclerosis (SPMS), in particular RRMS. These terms are defined below.

In an alternative embodiment of the present invention the patient to be treated does not meet one or more of the following criteria:
- being diagnosed with primary progressive multiple sclerosis or secondary progressive multiple sclerosis without disease activity
- meeting the criteria for neuromyelitis optica
- being lactating
- having an active, chronic immune system disease other than multiple sclerosis or having an immunodeficiency syndrome
- having neurological findings consistent with progressive multifocal leukoencephalopathy (PML) or confirmed PML
- having active systemic infections or acquired immunodeficiency syndrome or testing positive for human immunodeficiency virus antibody at screening
- being at risk of developing or having reactivation of hepatitis, syphilis or tuberculosis
- having received any live or live attenuated vaccines during the 2 months before start of therapy.

Preferably, ofatumumab is administered parenterally, e.g. epidermal, intravenous, intramuscular, intraarterial, intrathecal, intracapsular, intraorbital, intracardiac, intradermal, intraperitoneal, intratendinous, transtracheal, subcutaneous, subcuticular, intraarticular, subcapsular, subarachnoid, intraspinal, intracranial, intrathoracic, epidural and intrasternal injection and infusion. The preferred route of administration is subcutaneous injection.

In one embodiment of the invention, an ofatumumab composition is formulated in accordance with routine procedures as a pharmaceutical composition adapted for intravenous administration to human beings. Typically, compositions for intravenous administration are solutions in sterile isotonic aqueous buffer. Where suitable, the composition may also include a solubilizing agent and a local anesthetic, such as lignocaine, to ease pain at the site of the injection. Generally, the ingredients are supplied either separately or mixed together in unit dosage form, for example, as a dry lyophilized powder, or water-free concentrate, in a hermetically sealed container, such as an ampoule or sachet, indicating the quantity of active agent.

Where the composition is to be administered by infusion, it can be dispensed with an infusion bottle containing sterile pharmaceutical grade water or saline.

Where the composition is administered by injection, an ampoule of sterile water for injection or saline can be provided so that the ingredients may be mixed prior to administration.

In one embodiment, a formulation for ofatumumab can be formulated according to a formulation disclosed in WO 2009/009407.

In one embodiment, ofatumumab is formulated in an antibody formulation wherein ofatumumab is present in an amount of about 20-300 mg/mL, 50-300 mg/mL, 100-300 mg/mL, 150-300 mg/mL, 200-300 mg/mL, or 250-300 mg/mL, preferably at 50 mg/ml.

In one embodiment, ofatumumab is formulated in an antibody formulation wherein the formulation comprises 10 to 100 mM sodium acetate, 25 to 100 mM sodium chloride, 0.5 to 5% arginine free base, 0.02 to 0.2 mM EDTA, 0.01 to 0.2% polysorbate 80, and adjusted to pH 5.0 to 7.0. Preferably the ofatumumab formulation comprises 50 mM sodium acetate, 51 mM sodium chloride, 1% arginine free base, 0.05 mM EDTA, 0.02% polysorbate 80, and adjusted to pH 5.5.

In one embodiment, the ofatumumab formulation is provided in a pre-filled syringe or in an auto-injector. Preferably a pre-filled auto-injector intended for the s.c. administration is used.

In a preferred embodiment, ofatumumab injection is a sterile, preservative-free solution for subcutaneous use. Preferably, each 20 mg/0.4 mL prefilled pen or prefilled syringe delivers 0.4 mL of solution. Preferably, each 0.4 mL contains 20 mg of ofatumumab and arginine (4 mg), disodium edetate (0.007 mg), polysorbate 80 (0.08 mg), sodium acetate trihydrate (2.722 mg), sodium chloride (1.192 mg) and Water for Injection, USP with a pH of 5.5. Hydrochloric acid may be added to adjust pH.

In a preferred embodiment the ofatumumab formulation is intended for patient self-administration, preferably by subcutaneous injection.

In a preferred embodiment said formulation is administered in the abdomen, thigh or outer upper arm subcutaneously. In a preferred embodiment said formulation is not administered into moles, scars or areas where the skin is tender, bruised, red, hard or not intact.

In an embodiment, the first injection of said ofatumumab formulation may be performed under the guidance of a healthcare professional. If injection-related reactions occur, symptomatic treatment is recommended. Before administration, the pen or prefilled syringe is preferably removed from the refrigerator and allowed to reach room temperature, e.g. for about 15 to 30 minutes. In a preferred embodiment the ofatumumab formulation of the present invention is a clear to slightly opalescent and colorless to slightly brownish-yellow solution available as follows:
- Injection: 20 mg/0.4 mL in a single-dose prefilled pen, e.g. Sensoready^{®} pen
- Injection: 20 mg/0.4 mL in a single-dose prefilled syringe.

In a preferred embodiment, a subcutaneous ofatumumab dose of 20 mg every 4 weeks leads to a mean AUCₜₐᵤ of about 400 to 550, more preferably 450 to 500, e.g. 483 mcg h/mL and/or to a mean Cₘₐₓ of 1.0 to 2.5, more preferably 1.2 to 1.7, e.g. 1.43 mcg/mL at steady state. In a preferred embodiment, the volume of distribution at steady-state can be 4.5 to 6.5, more preferably 5.0 to 6.0, e.g. 5.42 L following subcutaneous administration of repeated ofatumumab 20 mg doses.

After subcutaneous administration, ofatumumab can be absorbed via the lymphatic system.

### BRIEF DESCRIPTION OF THE DRAWINGS

FIGURE 1 shows patient exhibited significant Expanded Disability Status Scale (EDSS) progression after administration of ocrelizumab despite immune reconstitution more than 3 months after fingolimod withdrawal.
FIGURE 2 shows Ofatumumab-induced CDC of freshly isolated primary human B cells
FIGURE 3 shows Ofatumumab Potently Induces CDC after Delayed Addition of Complement

### DEFINITIONS

### Antibody:

The term "antibody" as used herein refers to an immunoglobulin molecule, a fragment of an immunoglobulin molecule, or a derivative of either thereof, which has the ability to specifically bind to an antigen. The binding is preferably under typical physiological conditions for a significant period of time. The term "antigen-binding portion" of an antibody as used herein, refers to fragments of an antibody that retain the ability to specifically bind to an antigen (e.g., CD20). With regard to further definitions reference is made to WO 2018/033841, in particular pages 9-13.

### Breakthrough disease

For the purpose of this invention, breakthrough disease is defined as:
at least one documented relapse during the previous year OR two relapses during the previous two years,
presence of at least one Gd+ lesion on an MRI scan within the last 12 months, and/or presence of new or enlarging T2 lesions within the last 12 months.

### CD20:

The CD20 molecule (also called human B-lymphocyte-restricted differentiation antigen or Bp35) is a hydrophobic transmembrane protein with a molecular weight of approximately 35 kD located on pre-B and mature B lymphocytes. CD20 is found on the surface of more than 90% of B cells from peripheral blood or lymphoid organs and is expressed during early pre-B cell development and remains until plasma cell differentiation. CD20 is present on both normal B cells as well as malignant B cells. The 85 amino acid carboxyl-terminal region of the CD20 protein is located within the cytoplasm. Reference is made to the description in the GenBank, Accession No. NP_690605.

### Disease-modifying therapy (DMT)

The term "disease-modifying therapy" is used because there is still no curative treatment for multiple sclerosis (MS), but several disease-modifying drugs (DMDs) have been approved for MS. Generally, DMTs for RMS decrease the frequency and/or seriousness of relapses. Thus, DMTs are not a cure for RMS patients, but they can reduce how many relapses someone has and how serious they are.

### EEDS

The Expanded Disability Status Scale (EDSS) is a method of quantifying disability in multiple sclerosis and monitoring changes in the level of disability over time.

The EDSS scale ranges from 0 to 10 in 0.5 unit increments that represent higher levels of disability. Scoring is based on an examination by a neurologist.

EDSS steps 1.0 to 4.5 refer to people with MS who are able to walk without any aid and is based on measures of impairment in eight functional systems (FS):
- pyramidal - muscle weakness or difficulty moving limbs
- cerebellar - ataxia, loss of balance, coordination or tremor
- brainstem - problems with speech, swallowing and nystagmus
- sensory - numbness or loss of sensations
- bowel and bladder function
- visual function - problems with sight
- cerebral functions - problems with thinking and memory
- other.

A functional system (FS) represents a network of neurons in the brain with responsibility for particular tasks. Each FS is scored on a scale of 0 (no disability) to 5 or 6 (more severe disability). Reference is made to Kurtzke JF. Rating Neurologic Impairment in Multiple Sclerosis: An Expanded Disability Status Sclale (EDSS). Neurology. 1983, Nov;33(11):1444-52.

### Gd+ lesion

Gadolinium ("contrast") is a chemical compound that is injected into a person's vein during an MRI scan. Gadolinium normally cannot pass from the bloodstream into the brain or spinal cord due to the blood-brain barrier. But during active inflammation within the brain or spinal cord, as during an MS relapse, the blood-brain barrier is disrupted, allowing gadolinium to pass through. Gadolinium can then enter the brain or spinal cord and leak into an MS lesion, lighting it up, and creating a highlighted spot on an MRI. Such an MS lesion is called gadolinium-enhanced lesion or Gd+ lesion.

### Half-life

The half-life of a biological substance (e.g. a DMT's medication) is the time it takes for half of said substance to be removed by biological processes. This concept is used when the rate of removal is roughly exponential. In a medical context, half-life explicitly describes the time it takes for the blood plasma concentration of a substance to halve (plasma half-life) its steady-state when circulating in the full blood of an organism.

### Loading dose

A loading dose is an initial dose of a drug, preferably an initial higher dose, that may be given at the beginning of a course of treatment (e.g. a DMT) before succeeding with a maintenance dose, preferably dropping down to a lower maintenance dose.

### Neurologically stable

A clinical state characterized by lack of change in mental status or level of consciousness. This state may comprise control of seizures; absence of new neurologic defects, e.g. aphasia, ataxia, dysarthria, paresis, paralysis, visual field loss, or blindness, and is defined as neurologically stability.

### Multiple Sclerosis Impact Scale (MSIS-29)

The MSIS-29 version 2 is a 29-item, self-administered questionnaire that includes 2 domains: physical and psychological. Responses were captured on a 4-point ordinal scale ranging from 1 (not at all) to 4 (extremely), with higher scores reflecting greater impact on day-to-day life. The MSIS-29 takes about 5 minutes to complete and the questions are designed to determine the patient's views about the impact of MS on their day-to-day life during the past 2 weeks. Reference is made to Hobart J and Cano S (2009), "Improving the evaluation of therapeutic interventions in multiple sclerosis: the role of new psychometric methods", Health Technol Assess; 13(12):iii, ix-x, 1-177. NS RO to Hobart J, Lamping D, Fitzpatrick R, et al (2001), "The Multiple Sclerosis Impact Scale (MSIS-29): a new patient-based outcome measure", Brain; 124(Pt 5):962-73.

### Ofatumumab:

Ofatumumab is a human monoclonal antibody for the CD20 protein. Ofatumumab may bind specifically to both the small and large extracellular loops of the CD20 molecule. The Fab domain of ofatumumab may bind to the CD20 molecule and the Fc domain mediates immune effector functions to result in B-cell lysis in vitro. In particular, ofatumumab is a recombinant human monoclonal immunoglobulin G1 (IgG1) antibody that binds to human CD20 expressed on e.g. B cells. Ofatumumab is produced in a murine NS0 cell line and consists of two IgG1 heavy chains and two kappa light chains with a molecular weight of approximately 146 kDa.

Ofatumumab is described in EP 1 558 648 B1 and EP 3 284 753 B1. Further reference is made to the description in the drugbank.ca, accession number DB06650 and to WHO Drug Information, Vol. 20, No. 1, 2006. In an embodiment, the protein chemical formula is C₆₄₈₀H₁₀₀₂₂N₁₇₄₂O₂₀₂₀S₄₄ and the protein average weight is about 146100 Da.

The metabolic pathway of ofatumumab can be degradation to small peptides and amino acids by ubiquitous proteolytic enzymes. Ofatumumab might be eliminated in two ways: a target-independent route as with other IgG molecules and a target-mediated route that is related to binding to B cells.

The half-life of ofatumumab at steady state can be approximately 16 days, in particular following subcutaneous administration of repeated 20 mg doses.

Ofatumumab preferably does not share a common clearance pathway with chemical drugs that are metabolized by the cytochrome P450 system or other drug metabolizing enzymes. Preferably, ofatumumab is not involved in the regulation of the expression of drug metabolizing enzymes.

### Patient

The term "patient" preferably refers to a human patient, preferably an adult.

### Rebound

Severe disease reactivation after the withdrawal of DMT that exceeds a patient's pre-DMT baseline is considered a rebound event. Reference is made to Barry et al., Fingolimod Rebound: A Review of the Clinical Experience and Management Considerations. Neurol Ther (2019) 8:241-250.

### Relapse

Relapses can be defined as a new neurologic deficit or episode of neurologic worsening, preferably lasting longer than 24 h. In other words, relapses can be regarded as discrete episodes (in the art also referred to as "attacks," "flare-ups," or "exacerbations") of neurologic dysfunction, preferably lasting at least 24 h. Usually, relapses are followed by full or partial recovery and a period in which there is no symptom progression or accumulation of disability (remission).

Relapses are presumed to be caused by a new or enlarging demyelinating plaque at the site of an inflammatory event within the central nervous system (CNS).

### Revised McDonald criteria (Thompson et al 2018)

Under the revised McDonald Criteria, an MS diagnosis is likely if myelin damage is disseminated in space (DIS), as seen in an MRI as:
- At least one T2 bright lesion in at least two or four CNS locations: the juxtacortical, perventricular and infratentorial areas of the brain, and the spinal cord. (T2 is the most common MRI scan used to diagnose MS and to detect areas of myelin damage, old and new, in the brain and spinal cord).
- These lesions need not be gadolinium enhanced (contrast material).

Regarding myelin damage dissemination in time (DIT), MRI evidence is:
- A new T2 and/or gadolinium-enhancing damage on follow-up MRI, compared to a baseline scan (irrespective of time since baseline). The 2005 revision had required at least 30 days to pass between the initial and first attack.
- Simultaneous presence of asymptomatic gadolinium-enhancing and non-enhancing damage at any time.

Progressive primary MS (PPMS) has special diagnostic needs. Specifically, the revised McDonald Criteria calls for at least one year of demonstrated progression (done prospectively or retrospectively), plus two of the following three findings:
- Evidence of DIS in the brain, seen in at least one T2 lesion in the three key brain regions (periventricular, juxtacortical or infratentorial)
- Evidence of DIS in the spinal cord, based on at least two T2 lesions DIS (≥ 2 T2 damage);
- Positive CSF involvement, again as seen in the presence of oligoclonal bands and/or a high IgG index.

### RRMS

Relapsing-remitting multiple sclerosis (MS) is characterized by relapses, e.g. defined as a new neurologic deficit or episode of neurologic worsening lasting longer than 24 h, often in the absence of fever or infection.

There is no apparent progression of the disease during the periods of remission. At different points in time, RRMS can be further characterized as either active (with relapses and/or evidence of new MRI activity) or not active, as well as worsening (a confirmed increase in disability over a specified period of time following a relapse) or not worsening. Reference is made to Lublin 2014, Neurology. 2014 Jul 15; 83(3): 278-286.

### RMS

The term RMS (relapsing multiple sclerosis) encompasses RRMS, SPMS and clinically isolated syndrome (CIS).

### Primary progressive MS (PPMS)

PPMS is characterized by worsening neurologic function (accumulation of disability) from the onset of symptoms, without early relapses or remissions. PPMS can be further characterized at different points in time as either active (with an occasional relapse and/or evidence of new MRI activity) or not active, as well as with progression (evidence of disease worsening on an objective measure of change over time, with or without relapse or new MRI activity) or without progression. References is made to Lublin 2014.

Each person's experience with PPMS will be unique. PPMS can have brief periods when the disease is stable, with or without a relapse or new MRI activity, as well as periods when increasing disability occurs with or without new relapses or lesions on MRI.

### Secondary progressive MS (SPMS)

SPMS follows an initial relapsing-remitting course. Most people who are diagnosed with RRMS will eventually transition to a secondary progressive course in which there is a progressive worsening of neurologic function (accumulation of disability) over time. SPMS can be further characterized at different points in time as either active (with relapses and/or evidence of new MRI activity) or not active, as well as with progression (evidence of disease worsening on an objective measure of change over time, with or without relapses) or without progression. Reference is made to Lublin 2014.

Each person's experience with SPMS will be unique. SPMS follows after relapsing-remitting MS. Disability gradually increases over time, with or without evidence of disease activity (relapses or changes on MRI). In SPMS, occasional relapses may occur, as well as periods of stability.

### Clinically isolated syndrome (CIS):

Clinically isolated syndrome (CIS) may refer to a single clinical attack of central nervous system (CNS) inflammatory demyelinating symptoms that are suggestive of multiple sclerosis (MS). CIS presentations can be monofocal or multifocal and typically may involve the optic nerve, brainstem, cerebellum, spinal cord or cerebral hemispheres. Reference is made to Miller et al, Clinically isolated syndromes, Lancet Neurol. 2012;11:157-169.

### T1 and T2 lesions

T1 and T2 relate to different MRI methods used to generate magnetic resonance images. Specifically, T1 and T2 refers to the time taken between magnetic pulses and recording of an image. These different methods are used to detect different structures or chemicals in the central nervous system. T1 and T2 lesions refers to whether the lesions were detected using either the T1 or T2 method. A T1 MRI image supplies information about current disease activity by highlighting areas of active inflammation. A T2 MRI image provides information about disease burden or lesion load (the total amount of lesion area, both old and new).

### Wash-out

The term wash out relates to a period between clinical treatments (preferably periods between different DMTs) in which any medication delivered as the first treatment (e.g. first DMT) is allowed to - partly or fully - wash out of a patient before the second treatment (e.g. second DMT) begins. During the washout period, the patient preferably does not receive any other medication (e.g. DMT for the treatment of MS). Preferably, the medication is a disease modifying drug (DMD).

In a preferred embodiment, the medication of the first DMT is considered to be washed out if 25%, preferably 50%, more preferably 75%, even more preferred 85%, most preferred 95% of the medication's half-life have elapsed until the last dose of the first DMT's medication has been administrated before discontinuation of the first DMT.

In an alternative embodiment, the first DMT's medication is considered to be washed out if only 30%, preferably 20%, more preferably 10%, even more preferred 5%, most preferred 2.5% or less of the amount administered as the last dose of the first DMT's medication can be detected in samples (e.g. blood or serum) from the patient. In a particularly preferred embodiment, said amount can be replaced by Cₘₐₓ, i.e. the maximum (or peak) serum concentration that the first DMT's medication achieves in serum after the last dose of the first DMT's medication has been administrated before discontinuation of the first DMT.

Detection of the first DMT may be performed by one or more of PAGE, Western blot, ELISA, HPLC and mass spectrometry, capillary electrophoresis, Fourier transform infrared spectroscopy, circular dichroism, DLS, thermoshift assay, NMR, X-ray, chromatography and fluorescence spectroscopy.

### EXAMPLES

### Example 1:

### In-vitro data show differences between anti-CD20 antibodies with regard to induction of Complement-Dependent Cytotoxicity (CDC)

In vivo, anti-CD20 antibodies may mediate B cell killing by cellular and/or complement-dependent mechanisms. CDC is an important mechanism of ofatumumab-induced B cell lysis.

PBMCs (peripheral blood mononuclear cells) were prepared and human primary B cells were harvested by centrifugation and re-suspended at 2×10⁵ cells/mL in assay medium (RPMI 1640 + Glutamax supplemented with 0.1% BSA and 20 mM HEPES. Then, B cells were incubated in v-bottom 96 well plates simultaneously with antibody at a 3-fold serial dilution and human serum (30%) as a source of complement at 37°C, 5% CO₂ for 1 hour. Cells were washed, stained with SYTOX Blue (0.25 µM) and then analyzed for B cell lysis by flow cytometry on a FACS Fortessa. Cell death was defined as SYTOX Blue⁺ cells. Results are shown in Figure 2 demonstrating the strong concentration-dependent induction by ofatumumab of CDC in primary human B cells as compared to ocrelizumab and rituximab.

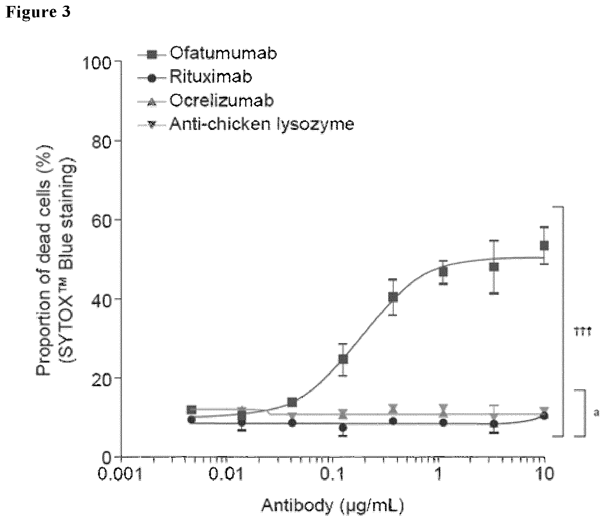

In a second experiment, B cells were incubated with antibody as described before, albeit in the absence of a source of complement. Cells were washed as described previously. After 6 hours, the source of complement was added. Although only low off-rate antibodies stay bound to cells and bind complement, ofatumumab potently induced CDC after this delayed addition of complement. This is shown in Figure 3.

Figure 2: Ofatumumab-induced CDC of freshly isolated primary human B cells

Figure 3: Ofatumumab Potently Induces CDC after Delayed Addition of Complement

Thus, surprisingly, ofatumumab provides the ability to activate complement factors and to induce CDC. In terms of potency and efficiency, ofatumumab is clearly distinguished from ocrelizumab in this respect.

### Example 2:

### Patient data showing reduction in relapses and Gd+ lesions after switch

The effect of ofatumumab administered to patients who have been treated with DMTs other than ofatumumab has been investigated as described below.

### 1. Ofatumumab

Ofatumumab was provided in pre-filled syringes for subcutaneous administration containing 20 mg ofatumumab (50 mg/ml, 0.4 ml content). The matching placebo to ofatumumab pre-filled syringe had the same appearance as the investigational drug.

Control treatment can be teriflunomide (Aubagio^{®}) 14 mg.

Ofatumumab arm: ofatumumab 20 mg s.c. injections on Day 1, 7, 14, Week 4 (Study Month 1) and every 4 weeks thereafter + teriflunomide-matching placebo capsule orally once daily.

Eligible patients can be randomized in a 1:1 ratio to either the active ofatumumab 20 mg group or to the active teriflunomide 14 mg group. The randomization was stratified by geographical region and by MS subtype (RRMS, SPMS).

### 2. Patient population

Patients eligible for inclusion in this study had to fulfill the following criteria:
Diagnosis of MS according to the 2010 Revised McDonald criteria (Polman et al. 2011).
Relapsing MS: relapsing-remitting course (RRMS), or secondary progressive (SPMS) course with disease activity, as defined by Lublin et al., 2014.

Disability status at Screening with an EDSS score of 0 to 5.5 (inclusive).

Patients previously treated with DMT selected from glatiramer acetate, dimethyl fumarate (DMF), daclizumab, fingolimod, natalizumab and laquinimod.

Documentation of at least: 1 relapse during the previous 1 year OR 2 relapses during the previous 2 years prior to Screening OR a positive Gd-enhancing MRI scan during the year prior to randomization. Note: Screening MRI scan may be used if no positive Gd enhancing scan exist from prior year.

Neurologically stable within 1 month prior to randomization

### 3. Results

### 3.1 Results for patients being treated with fingolimod as earlier DMT

### a) Unexpected drop of relapses

The following table shows the results of a first study for the number of relapses before enrolling the study and after (on-study by month 12 and month 24).

| Drug | Obs Variable | N | Mean |
|---|---|---|---|
| OMB | NURLP12 | 39 | 1.26 |
| | NURLP24a | 39 | 2.28 |
| | NCFRLP12 | 39 | 0.23 |
| | NCFRLP24 | 39 | 0.31 |

In the above Table, the following abbreviations are used:

| Abbreviation | Explanation |
|---|---|
| N | N: number of evaluable patients |
| NURLP 12 | number of relapses in past 12 months (screening) |
| NURLP24a | number of relapses in past 24 months (screening) |
| NCFRLP12 | number of relapses up to month 12 post-first dose |
| NCFRLP24 | number of relapses up to month 24 post-first dose |

The table shows that, among patients randomized to ofatumumab (Study 1), 27 patients were evaluated at each visit, i.e. baseline, 12 months and 24 months. The number of relapses was approx. 1.26 per patient in the 12 months preceding screening. This rate unexpectedly dropped to approx. 0.23 in the 12 months following treatment switch and to about 0.30 in the 24-month period.

The drop in the ofatumumab group was unexpectedly large. The drop was larger than in the comparative group.

### b) Unexpected reduction of Gd-enhancing T1 lesions

The following results were obtained for the Gd lesion numbers.

| Drug | Analysis Visit | N | Mean |
|---|---|---|---|
| OMB | BASELINE | 37 | 2.95 |
| | Week 48 | 36 | 0.03 |
| | Week 96 | 27 | 0 |

The table shows that, among patients randomized to ofatumumab surprisingly the number of Gd+ lesions were surprisingly reduced.

### 3.2 Results for patients being treated with DMF as earlier DMT

| Drug | Obs Variable | N | Mean |
|---|---|---|---|
| OMB | NURLP12 | 56 | 1.11 |
| | NURLP24a | 56 | 2 |
| | NCFRLP12 | 56 | 0.21 |
| | NCFRLP24 | 56 | 0.39 |

| Drug | Analysis Visit | N | Mean |
|---|---|---|---|
| OMB | BASELINE | 55 | 2.02 |
| | Week 48 | 52 | 0 |
| | Week 96 | 33 | 0 |

### 3.3 Results for patients being treated with daclizumab as earlier DMT

| Drug | Obs Variable | N | Mean |
|---|---|---|---|
| OMB | NURLP12 | 11 | 1 |
| | NURLP24a | 11 | 1.18 |
| | NCFRLP12 | 11 | 0 |
| | NCFRLP24 | 11 | 0 |

| Drug | Analysis Visit | N | Mean |
|---|---|---|---|
| OMB | BASELINE | 11 | 0.18 |
| | Week 48 | 10 | 0 |
| | Week 96 | 2 | 0 |

### 3.4 Results for patients being treated with natalizumab as earlier DMT

| Drug | Obs Variable | N | Mean |
|---|---|---|---|
| OMB | NURLP12 | 30 | 1.2 |
| | NURLP24a | 30 | 2.37 |
| | NCFRLP12 | 30 | 0.13 |
| | NCFRLP24 | 30 | 0.2 |

| Drug | Analysis Visit | N | Mean |
|---|---|---|---|
| OMB | BASELINE | 28 | 2.5 |
| | Week 48 | 27 | 0.04 |
| | Week 96 | 20 | 0 |

### 4.5 Results for patients being treated with glatiramer acetate as earlier DMT

| Drug | Obs Variable | N | Mean |
|---|---|---|---|
| OMB | NURLP12 | 156 | 1.21 |
| | NURLP24a | 156 | 2.13 |
| | NCFRLP12 | 156 | 0.10 |
| | NCFRLP24 | 156 | 0.17 |

| Drug | Analysis Visit | N | Mean |
|---|---|---|---|
| OMB | BASELINE | 154 | 1.52 |
| | Week 48 | 147 | 0.02 |
| | Week 96 | 128 | 0.02 |

### Example 3:

### Clinical trial

The results as described above in example 2 are confirmed in the clinical trial as described below.

### 1. Population

The study population consists of adult subjects with RMS. The study is conducted in approximately 120-170 centers worldwide.

Subjects eligible for inclusion in this study must meet all of the following criteria:
1. Diagnosis of MS according to the 2017 Revised McDonald criteria
2. Relapsing MS: relapsing forms of MS (RMS) including RMS and secondary progressive MS (SPMS).
3. Disability status at Screening with an EDSS score of 0 to 4 (inclusive).
4. MS treatment history with a maximum of 3 DMTs
5. Subject transitioning from either fingolimod or dimethyl fumarate which was administered for a period of at least 6 months, as their last DMT before first study drug administration
6. Breakthrough disease activity while the participant was adequately using fingolimod or dimethyl fumarate prior to transitioning as evidenced by one or more clinically reported relapses or one or more signs of MRI activity (e.g. Gd+ enhancement, new or enlarging T2 lesions)
7. Neurologically stable within one month prior to first study drug administration.

### 2. Treatment drug, treatment arms, treatment duration

Ofatumumab is provided in an autoinjector (AI) for subcutaneous administration containing 20 mg ofatumumab (50 mg/ml, 0.4 ml content). Ofatumumab (is clear to opalescent, colorless to pale yellow, essentially particle-free liquid.

This is an open label treatment study with one arm.

The planned duration of treatment is 96 weeks.

### 3. Transition period preferably including washout from previous DMT

For the purpose of this study, we define the transitioning period as the time between stopping the current treatment (fingolimod or DMF) and starting ofatumumab treatment. The exact timing of the transitioning is based on the clinical judgment of the investigator.

During a washout period, the subject may not receive any other DMT for the treatment of MS.

### 4. Instruction for prescribing and taking study treatment

The study drug (ofatumumab injections) is administered starting at Visit 1. Drug is then dispensed at scheduled visits throughout the treatment period. Starting at week 4, the subcutaneous injections should be administered at 4-week intervals (+/- 3 days).

In order to assess tolerability of the initial dose of study medication, subjects are closely monitored following administration for any reactions including injection related ones.

At Visit 1, subjects receive the s.c. injection at site. The subject or a caregiver injects the study medication under supervision of the study staff.

Following Visit 1, subjects may inject the study medication at home by themselves or have a caregiver who has been trained by the study staff on the proper technique and safety precautions, inject the study medication. Ability to self-administer injections must be demonstrated and documented before home-administration is permitted. Injection 2 (Day 7) is overseen remotely by the designated site personnel to support the self/home administration and provide additional training as needed. Subjects return to the site for the week 2 and 4 administrations.

### Example 4: Clinical trial showing reduced thalamus volume loss and improved MSIS-29

### Background

Ofatumumab demonstrated superior efficacy versus teriflunomide in Phase 3 ASCLEPIOS I/II trials, see ECTRIMS Online Library. Hauser S. et al. 09/13/19; 279581; 336. MS patients on ofatumumab had a reduction in annualized relapse rate (ARR) by 50.5% (0.11 vs. 0.22) and 58.5% (0.10 vs. 0.25) compared to Aubagio^{®} (teriflunomide) (both studies p<0.001) in ASCLEPIOS I and II studies respectively. Ofatumumab showed a highly significant suppression of gadolinium (Gd) T1 lesions when compared to Aubagio^{®}, demonstrating a profound suppression of new inflammatory activity. Ofatumumab showed a relative risk reduction of 34.4% in 3-month confirmed disability progression (CDP) (p=0.002) and 32.5% in 6-month CDP (p=0.012) versus Aubagio^{®} in pre-specified pooled analyses.

### Objectives and Methods

In ASCLEPIOS I patients were randomised (1:1) to receive either ofatumumab 20 mg sc injections every 4 weeks (after an initial loading regimen of 20 mg sc doses on Days 1, 7 and 14) or teriflunomide 14 mg orally once daily, for up to 30 months. Patients aged 18-55 years with an Expanded Disability Status Scale (EDSS) score (according to Kurtzke, Neurology. 1983, Nov; 33(11): 1444-52) of 0-5.5 at screening who experienced ≥1 relapse in the past year or ≥2 relapses in the past 2 years or a positive gadolinium-enhancing (Gd+) MRI scan during the year before randomisation were included.

### Results

### (i) Thalamus volume loss

Ofatumumab compared with teriflunomide significantly reduced the loss of thalamus volume between Month 24 and baseline (mean percentage change -1.00 vs -1.40, mean difference 0.40, p=0.002).

### (ii) MSIS-29

A higher score on the MSIS-29 is indicative of a greater impact of MS on the daily life from a patient's perspective. Ofatumumab treatment reduced the impact of MS on the patient's daily life compared with teriflunomide: the mean change in MSIS-29 physical impact score from baseline was significantly greater in the ofatumumab group than in the teriflunomide treatment group at all time points i.e. at Month 6 (-2.75 vs -0.44, mean difference -2.30, p=0.017), at Month 12 (-2.43 vs 0.17, mean difference -2.59, p=0.009), Month 18 (-2.37 vs 0.67, mean difference -3.05, p=0.005), Month 24 (-2.6 vs 0.59, mean difference -3.19, p=0.008) and Month 30 (-3.21 vs 0.55, mean difference -3.76, p=0.026) (Table 14.2-7.1).

Ofatumumab treatment was associated with a stronger reduction on the impact of MS on the patient's daily life vs teriflunomide treatment group as measured by the MSIS-29 psychological impact score. However, the difference between treatment groups only reached statistical significance at Month 12.

### Conclusions

(i) Thalmic volume is a MRIbased marker associated with neurodegeneration to hasten development of neuroprotective treatment. According to the prior art thalamic volume declined in MS subjects with an estimated decline of-0.71% per year and -0.28% per year in healthy controls, see Azevedo 2018.Treatment with teriflunomide(- 1.4 % in 2 years) does not seem to have a beneficial effect re loss of thalmic volume when compared to the data available of the prior art. Contrary, switching to ofatumumab as disease-modifying treatment shows promising results.
(ii) Analysis of proportion of patients free of clinical and MRI disease activity (i.e. patients with NEDA-4) and the health-related quality of life measure MSIS-29 showed beneficial effects and were supportive in demonstrating the robustness of the treatment effect of ofatumumab.

### Example 5: Maintained ofatumumab efficacy in relapsing MS patients who transition from intravenous anti-CD20 therapy

Background: Depletion of B cells in patients with relapsing multiple sclerosis (RMS) using anti-CD20 monoclonal antibodies (mAbs) reduces annualized relapse rates and inflammatory lesion activity on magnetic resonance imaging and delays time to confirmed disability worsening. Anti-CD20 mAbs ocrelizumab and rituximab are administered by intravenous infusion in clinic; ofatumumab is administered subcutaneously with a pre-filled syringe or autoinjector (AI) pen, facilitating self-administration.

Objectives: 12-month, prospective, single-arm, multicenter clinical trial that confirms maintained efficacy of ofatumumab in patients with RMS who transition from intravenous anti-CD20 mAb therapy.

Methods: About 100 adults with RMS are enrolled at 10-20 centers in the USA. Eligible patients have been previously treated with 2-5 consecutive courses of intravenous ocrelizumab or rituximab (other anti-CD20 mAbs are excluded), with a last dose 4-9 months before baseline. Other inclusion criteria are Expanded Disability Status Scale score 5.5 or lower at screening and CD19 B cells depleted to below 1% of anti-CD20 therapy baseline. Patients with suboptimal response to anti-CD20 therapy in the previous 6 months (relapse, ≥2 active gadolinium-enhancing [Gd+] lesions, any new/enlarging T2 lesions, clinical worsening), or who discontinued anti-CD20 therapy because of severe infusion-related reactions or recurrent infections, or with progressive disease, are excluded. All participants receive subcutaneous ofatumumab 20 mg administered by AI pen on Days 1, 7 and 14, then monthly in Months 1-12. The primary endpoint is no change or a reduction in Gd+ lesion count at Month 12. Secondary endpoints are participant retention and changes in immune biomarkers, treatment satisfaction, safety and tolerability at Months 6 and 12. There is a 6-month interim analysis.

Results: The trial complements the ofatumumab phase 3 program in RMS by generating maintained efficacy, retention and satisfaction data based on monthly subcutaneous drug delivery with the AI pen in patients previously treated with ocrelizumab or rituximab.

Conclusions: The trial provides important data on the maintained efficacy of ofatumumab in patients with RMS transitioning from intravenous anti-CD20 therapies.

### Further numbered embodiments

1. Ofatumumab for use in the treatment or prevention of relapsing multiple sclerosis, wherein ofatumumab is used in a patient who has been treated with a disease-modifying therapy other than ofatumumab.
2. Ofatumumab for use according to embodiment 1, wherein the disease-modifying therapy lacks efficacy.
3. Ofatumumab for use according to any one of the preceding embodiments, wherein the patient develops breakthrough disease due to treatment with the disease-modifying therapy.
4. Ofatumumab for use according to embodiment 3, wherein the breakthrough disease is evidenced by one or more clinically reported relapses or one or more signs of MRI activity.
5. Ofatumumab for use according to embodiment 4, wherein the MRI activity comprises Gd+ enhancement and/or new or enlarging T2 lesions.
6. Ofatumumab for use according to any one of the preceding embodiments, wherein the patient lacks tolerability for the disease-modifying therapy.
7. Ofatumumab for use according to any one of the preceding embodiments, wherein the patient has a history of at most 1 or 2 or 3 disease-modifying therapies.
8. Ofatumumab for use according to any one of the preceding embodiments, wherein the disease-modifying therapy was administered for a period of at least 6 months before the first administration of ofatumumab.
9. Ofatumumab for use according to any one of the preceding embodiments, wherein the patient is neurologically stable within one month prior to the first administration of ofatumumab.
10. Ofatumumab for use according to any one of the preceding embodiments, wherein the patient has an EDSS score of 1 to 4 prior to the first administration of ofatumumab.
11. Ofatumumab for use according to any one of the preceding embodiments, wherein the earlier disease-modifying therapy is administered orally.
12. Ofatumumab for use according to any one of the preceding embodiments, wherein the drug of the earlier disease-modifying therapy is selected from teriflunomide, dimethyl fumarate and fingolimod.
13. Ofatumumab for use according to any one of embodiments 11 or 12, wherein the earlier disease-modifying therapy is fingolimod.
14. Ofatumumab for use according to embodiment 13, wherein fingolimod was administered in a daily dose of 0.5 mg.
15. Ofatumumab for use according to any one of embodiments 1 to 10, wherein the drug of the earlier disease-modifying therapy is selected from natalizumab, rituximab, ocrelizumab, alemtuzumab, daclizumab and glatirameracetat.
16. Ofatumumab for use according to any one of embodiments 1 to 10 and 15, wherein the earlier disease-modifying therapy is selected from intravenous anti-CD20 therapies.
17 Ofatumumab for use according to embodiment 16, wherein ofatumumab is administered to patients with suboptimal response to anti-CD20 therapy, preferably to patients with suboptimal response in the previous 6 months.
18. Ofatumumab for use according to embodiment 16 or 17, wherein ofatumumab is administered to patients with adverse events to anti-CD20 therapy, in particular infusion-related reactions or recurrent infections.
19. Ofatumumab for use according to any one of embodiments 16 to 18, wherein patients have been previously treated with at least 2 consecutive courses of intravenous ocrelizumab or rituximab.
20. Ofatumumab for use according to any one of embodiments 16 to 19, wherein the last dose was administered 4-9 months before ofatumumab was administered.
21. Ofatumumab for use according to any one of the preceding embodiments, wherein ofatumumab is administered after an relapse.
22. Ofatumumab for use according to any one of the preceding embodiments, wherein ofatumumab is administered after the detection of at least one Gd+ lesion.
23. Ofatumumab for use according to any one of the preceding embodiments, wherein ofatumumab is administered after the detection of new or enlarging T2 lesions.
24. Ofatumumab for use according to any one of the preceding embodiments, wherein the earlier disease-modifying therapy is discontinued before initiation of ofatumumab administration.
25. Ofatumumab for use according to embodiment 24, wherein discontinuation of the earlier disease-modifying therapy results in rebound.
26. Ofatumumab for use according any one of the preceding embodiments, wherein ofatumumab administration is started before the half-life of the drug used in the earlier disease-modifying therapy has been reached in the patient.
27. Ofatumumab for use according to any one of embodiments 1 to 26, wherein ofatumumab administration is started after a wash-out period of 1 day to 3 weeks.
28. Ofatumumab for use according to any one of embodiments 1 to 26, wherein ofatumumab administration is started without a wash-out period.
29. Ofatumumab for use according to any one of the preceding embodiments, wherein ofatumumab is administered if loss of thalamus volume was not sufficiently reduced by the earlier disease-modifying treatment.
30. Ofatumumab for use in the treatment or prevention of relapsing multiple sclerosis, wherein ofatumumab reduces the loss of thalamus volume, preferably below 0 0.65 % per year.
31. Ofatumumab for use according to any one of the preceding embodiments, wherein ofatumumab is administered if reduction of the MSIS-29 score was not sufficiently achieved by the earlier disease-modifying treatment.
32. Ofatumumab for use according to embodiment 31, wherein the MSIS-29 score was reduced by less than 2.5 by the earlier disease-modifying treatment.
33. Ofatumumab for use according to any one of the preceding embodiments, wherein ofatumumab is administered at a dose of 10 to 30 mg every 4 weeks, preferably 20 mg every 4 weeks.
34. Ofatumumab for use according to any one of the preceding embodiments, wherein ofatumumab is administered subcutaneously.
35. Ofatumumab for use according to any one of the preceding embodiments, wherein ofatumumab is administered with a loading dose.
36. Ofatumumab for use according to embodiment 35, wherein 20 mg ofatumumab at week 0, week 1 and week 2 is administered as a loading dose.
37. Ofatumumab for use according to any one of embodiments 1 to 34, wherein ofatumumab is administered without a loading dose.
38. Ofatumumab for use according to any one of the preceding embodiments, wherein relapsing multiple sclerosis is selected from relapsing remitting multiple sclerosis (RRMS), secondary progressive multiple sclerosis (SPMS) and clinically isolated syndrome.
39. Ofatumumab for use according to any one of the preceding embodiments, wherein a premedication is administered to the patient before the first dose of ofatumumab is administered.
40. Ofatumumab for use according to embodiment 39, wherein the premedication comprises acetaminophen, antihistamines and/or steroids.
41. Ofatumumab for use according to embodiment 39 or 40, wherein the premedication is administered 30 to 60 minutes prior to ofatumumab injection.
42. Ofatumumab for use according to any one of embodiments 1 to 38, wherein no premedication is administered prior to the first dose of ofatumumab.
43. Ofatumumab for use according to any one of the preceding embodiments, wherein rebound or recurrent disease activity is avoided.
44. Ofatumumab for us according to embodiment 2, wherein lack of efficacy is defined as not stopping or not appropriately slowing down disease progression.
45. Method for treating or preventing relapsing multiple sclerosis, wherein the method comprises administration of ofatumumab to a patient suffering from relapsing multiple sclerosis, said patient having taken a disease-modifying therapy other than ofatumumab.
46. Ofatumumab for producing a medicament for use in the treatment or prevention of relapsing multiple sclerosis, wherein the medicament is used in a patient who has been treated with a disease-modifying therapy other than ofatumumab.

## Claims

1. Ofatumumab for use in the treatment or prevention of relapsing multiple sclerosis, wherein ofatumumab is used in a patient who has been treated with a disease-modifying therapy other than ofatumumab, wherein the disease-modifying therapy lacks efficacy.

2. Ofatumumab for use according to claim 1, wherein the patient develops breakthrough disease due to treatment with the disease-modifying therapy,
optionally wherein the breakthrough disease is evidenced by one or more clinically reported relapses or one or more signs of MRI activity,
further optionally wherein the MRI activity comprises Gd+ enhancement and/or new or enlarging T2 lesions.

3. Ofatumumab for use according to any one of the preceding claims, wherein:
(a) the patient has a history of at most 1 or 2 or 3 disease-modifying therapies; and/or
(b) the disease-modifying therapy was administered for a period of at least 6 months before the first administration of ofatumumab.

4. Ofatumumab for use according to any one of the preceding claims, wherein the earlier disease-modifying therapy is selected from glatiramer acetate, dimethyl fumarate (DMF), daclizumab, fingolimod, natalizumab and laquinimod.

5. Ofatumumab for use according to any one of claims 1-3, wherein the earlier disease-modifying therapy is administered orally.

6. Ofatumumab for use according to any one of the claims 1-3 and 5, wherein the drug of the earlier disease-modifying therapy is selected from teriflunomide, dimethyl fumarate and fingolimod, optionally wherein:
(a) the earlier disease-modifying therapy is fingolimod; or
(b) the earlier disease-modifying therapy is dimethyl fumarate.

7. Ofatumumab for use according to any one of claims 1 to 3, wherein the drug of the earlier disease-modifying therapy is selected from natalizumab, rituximab, ocrelizumab, alemtuzumab, daclizumab and glatiramer acetate

8. Ofatumumab for use according to any one of claims 1 to 3 and 7, wherein the earlier disease-modifying therapy is selected from intravenous anti-CD20 therapies, optionally wherein:
(a) the patient has been previously treated with at least 2 consecutive courses of intravenous ocrelizumab or rituximab;
(b) the last dose was administered 4-9 months before ofatumumab was administered; and/or
(c) ofatumumab is administered subcutaneously.

9. Ofatumumab for use according to any one of the preceding claims, wherein:
(a) ofatumumab is administered after a relapse;
(b) ofatumumab is administered after the detection of at least one Gd+ lesion; and/or
(c) ofatumumab is administered after the detection of new or enlarging T2 lesions.

10. Ofatumumab for use according to any one of the preceding claims, wherein:
(a) the earlier disease-modifying therapy is discontinued before initiation of ofatumumab administration, optionally wherein discontinuation of the earlier disease-modifying therapy results in rebound; and/or
(b) ofatumumab administration is started before the half-life of the drug used in the earlier disease-modifying therapy has been reached in the patient.

11. Ofatumumab for use according to any one of the preceding claims, wherein:
(a) ofatumumab administration is started after a wash-out period of 1 day to 3 weeks; or
(b) ofatumumab administration is started without a wash-out period.

12. Ofatumumab for use according to any one of the preceding claims, wherein:
(a) ofatumumab is administered if loss of thalamus volume was not sufficiently reduced by the earlier disease-modifying treatment; and/or
(b) ofatumumab is administered if reduction of the MSIS-29 score was not sufficiently achieved by the earlier disease-modifying treatment, optionally wherein the MSIS-29 score was reduced by less than 2.5 by the earlier disease-modifying treatment.

13. Ofatumumab for use according to any one of the preceding claims, wherein:
(a) ofatumumab is administered at a dose of 10 to 30 mg every 4 weeks, preferably 20 mg every 4 weeks;
(b) ofatumumab is administered subcutaneously; and/or
(c) ofatumumab is administered with a loading dose, optionally wherein 20 mg ofatumumab at week 0, week 1 and week 2 is administered as a loading dose.

14. Ofatumumab for use according to any one of the preceding claims, wherein relapsing multiple sclerosis is selected from relapsing remitting multiple sclerosis (RRMS), secondary progressive multiple sclerosis (SPMS) and clinically isolated syndrome.

15. Ofatumumab for use according to any one of the preceding claims, wherein:
(a) rebound or recurrent disease activity is avoided; and/or
(b) lack of efficacy is defined as not stopping or not appropriately slowing down disease progression.
